# FASCICULE DE BREVET EUROPEEN

(11) **EP 2 429 590 B1**
(45) Date de publication et mention de la délivrance du brevet: **22.04.2020**
(21) Numéro de dépôt: 10720154.3
(22) Date de dépôt: 12.05.2010
(51) Int. Cl.: A61K 49/06

(54) **PROCÉDÉ DE PRÉPARATION D'UNE FORMULATION PHARMACEUTIQUE DE CHÉLATE DE LANTHANIDE**
VERFAHREN ZUR HERSTELLUNG EINER PHARMAZEUTISCHEN FORMULIERUNG AUS LANTHANIDCHELAT
METHOD FOR PREPARING A PHARMACEUTICAL FORMULATION OF LANTHANIDE CHELATE

(30) Priorité: 13.05.2009 FR 0953147
(43) Date de publication de la demande: 21.03.2012
(73) Titulaire: GUERBET, 93420 Villepinte (FR)
(72) Inventeur: PORT, Marc, F-95170 Deuil la Barre (FR)
(74) Mandataire: Regimbeau
(86) Numéro de dépôt international: PCT/EP2010/056603
(87) Numéro de publication internationale: WO 2010/130814

(56) Documents cités:
- EP-A- 0 270 483
- EP-A- 0 481 526
- WO-A-2009/103744
- FR-A- 2 590 484
- US-A- 4 647 447
- US-A- 5 650 133
- US-A1- 2004 170 566
- IDÉE JEAN-MARC ET AL: "Clinical and biological consequences of transmetallation induced by contrast agents for magnetic resonance imaging: a review." FUNDAMENTAL & CLINICAL PHARMACOLOGY DEC 2006, vol. 20, no. 6, décembre 2006 (2006-12), pages 563-576, XP002549063 ISSN: 0767-3981
- COROT C ET AL: "Structure-activity relationship of macrocyclic and linear gadolinium chelates: Investigation of transmetallation effect on the zinc-dependent metallopeptidase angiotensin-converting enzyme" JOURNAL OF MAGNETIC RESONANCE IMAGING, SOCIETY FOR MAGNETIC RESONANCE IMAGING, OAK BROOK, IL, US, vol. 8, no. 3, 1 mai 1998 (1998-05-01), pages 695-702, XP008097347 ISSN: 1053-1807
- "DOTAREM 0.5 mmol/ml, solution for injection vials, Pre-Filled Syringes", INTERNET CITATION, 1 June 2007 (2007-06-01), pages 1-4, XP003028305, Retrieved from the Internet: URL:http://www.health.gov.il/units/pharmac y/trufot/alonim/3768.pdf [retrieved on 2011-10-21]

## Description

L'invention concerne un procédé de préparation performant sur le plan industriel de formulations pharmaceutiques de chélates d'ions de métaux paramagnétiques, et l'utilisation de ces formulations pour la préparation d'agents de contraste pour l'Imagerie par Résonance Magnétique.

On connaît de nombreux agents de contraste à base de chélates de lanthanides (métal paramagnétique), en particulier de gadolinium, décrits par exemple dans le document US 4 647 447. Plusieurs produits sont commercialisés, notamment des chélates macrocycliques, tels que le gadotérate DOTA (acide 1,4,7,10-tétraazacyclododécane-N,N',N",N"'-tétraacétique) et le gadotéridol HPDO3A, et des chélates linéaires tels que le DTPA (acide diéthylènetriaminepentaacétique) et le DTPA-BMA (gadodiamide).

Dans l'organisme, les chélates de lanthanide sont en situation d'équilibre chimique, ce qui peut conduire à un risque de libération non souhaitée de lanthanide, et plus spécialement de gadolinium. L'homme du métier est ainsi amené à rechercher des solutions limitant ce risque pour résoudre de manière complètement sécurisée le problème complexe de la tolérance chez le patient. Ce problème est d'autant plus délicat que l'administration d'agents de contraste est souvent répétée lors d'examens de diagnostic et/ou pour le guidage et le suivi de l'efficacité d'un traitement thérapeutique.

Plusieurs axes d'amélioration de la tolérance de chélates de gadolinium sont décrits dans l'art antérieur. Il y a plus de vingt ans, notamment à travers le document US 5 876 695, l'homme du métier a travaillé sur des formulations comprenant outre le chélate complexant le lanthanide, un excès de chélate. Cet excès est destiné à compenser une libération non souhaitée du lanthanide, le chélate en excès venant complexer le lanthanide (ion métallique Gd3+) libéré. Le document US 5 876 695 décrit en particulier un excès de chélate linéaire, en particulier de DTPA libre. Les documents EP 450 078, US 5 876 695 et US 2004/0170566 notamment décrivent l'utilisation d'excipients s'écrivant sous la forme X[X',L] où X' est un cation métallique (notamment calcium) complexé par le chélate, et X est un cation métallique utilisé pour obtenir un sel non chargé du complexe [X',L] (chargé négativement sinon). X et X' sont typiquement choisis indépendamment l'un de l'autre parmi les ions sodium, zinc, magnésium et calcium, L représentant le chélate. Cet excipient permet donc de rajouter du chélate en excès.

Malgré toutes ces études de l'art antérieur, le problème complexe de la tolérance est toujours d'actualité, notamment dans des situations à risque de tolérance plus prononcé pour l'administration de produits de contraste IRM. Il est d'ailleurs apparu récemment un nouveau problème en matière de tolérance, à savoir une pathologie désignée NSF (nephrologic systemic fibrosis - fibrose néphrogénique systémique, ou dermopathie fibrogénique), qui pourrait être corrélée au moins en partie à l'existence de gadolinium dans l'organisme. Cette maladie a conduit à une alerte des autorités de santé vis-à-vis d'agents de contraste gadolinés commercialisés pour certaines catégories de patients.

En définitive ce problème de la tolérance de chélates de lanthanides reste complexe et important.

Le demandeur a montré dans la demande pendante FR0851055 l'importance et la difficulté d'obtenir à l'échelle industrielle pour des chélates macrocycliques, et en particulier le DOTA, des formulations comprenant un excès de chélate libre et dans une gamme de concentration étroite et précise [0,002 à 0,4%] mol/mol, et en particulier 0,025 à 0,25%. Par chélate libre il est fait référence à un chélate non complexé par un lanthanide ou un cation métallique, donc contrairement à l'art antérieur pas sous la forme d'un excipient de formule X[X',L] avec X, X' et L tels que définis ci-dessus. Pour résoudre ce problème le demandeur a réussi à mettre au point un procédé de préparation d'une formulation de chélate macrocyclique, de préférence le DOTA, le DO3A, le HPDO3A ou le PCTA, encore de préférence le DOTA, ledit procédé comprenant au moins une étape d'ajustement des quantités entre le chélate et le lanthanide de manière à obtenir dans la solution pharmaceutique finale (typiquement liquide injectée au patient), un excès de chélate mol/mol compris entre 0,002 et 0,4%, et plus spécialement entre 0,02 et 0,3%, très avantageusement entre 0,025 et 0,25%.

Le procédé décrit dans ce document comprend avantageusement les étapes suivantes :
1) mélange du chélate et du lanthanide pour obtenir la complexation du lanthanide par le chélate macrocyclique, typiquement à chaud (avantageusement entre 60 et 90 °C, de préférence autour de 80°C).
2) mesure des quantités du chélate et/ou du lanthanide libre dans la solution obtenue à l'étape 1)
3) ajustement des quantités de chélate et/ou de lanthanide.

Les étapes 2 et 3 se font par tout moyen approprié après avoir réalisé la complexation.

Typiquement on ajoute une quantité de chélate ou de lanthanide pour parvenir à la concentration cible en entités libres, typiquement en DOTA libre. On obtient ainsi à la fin de l'étape 3) une solution pharmaceutique ajustée typiquement sous forme liquide, comprenant le complexe, en particulier DOTA-Gd, et un excès de chélate libre, en particulier DOTA libre (0,1 à 0,2% par exemple).

A la fin de l'étape 3, soit cette formulation ajustée sous forme liquide est la solution injectée au patient (le cas échéant après ajout à cette solution d'excipients pharmaceutiques), soit cette formulation ajustée sous forme liquide est transformée (typiquement par évaporation, lyophilisation, concentration ou atomisation), en une poudre qui pourra être remise en solution (le cas échéant avec ajout à cette solution d'excipients pharmaceutiques) à injecter au patient.

Il est rappelé que de nombreuses variantes de réalisation sont inclues dans la portée de la demande pendante FR0851055.

Selon une de ces variantes la solution pharmaceutique ajustée obtenue après complexation (étape 1) ci-dessus) est additionnée de l'excipient de la formulation finale administrée au patient, cet excipient étant avantageusement la méglumine pour le DOTA-Gd. La solution pharmaceutique ajustée liquide alors obtenue est prête à être administrée au patient. Pour le DOTA par exemple, la solution administrée contient le complexe DOTA-Gd et un léger excès de DOTA libre. Selon une autre variante, cette solution pharmaceutique obtenue après complexation est transformée en poudre, de manière à obtenir cette fomulation ajustée sous forme d'une poudre. Pour le DOTA par exemple, cette poudre contient le complexe DOTA-Gd et le léger excès de DOTA libre ciblé. Si l'on a introduit l'excipient de formulation (méglumine par exemple) dès le début ou peu après la complexation, et avant la transformation en poudre, on obtient une poudre qui contient le complexe et l'excipient. On obtient ainsi en poudre, le sel de méglumine de l'acide gadotérique DOTA-Gd, cette poudre contenant le DOTA-Gd et du DOTA libre en excès dans la gamme de valeur ciblée.

Selon une autre variante la solution pharmaceutique ajustée issue de la complexation est transformée en matériau de type poudre, par tout procédé approprié, cette poudre étant ensuite remise en solution avec d'une part un éventuel nouvel ajustement par ajout à cette solution de DOTA libre par exemple, et d'autre part un ajout de l'excipient de formulation, cet excipient étant avantageusement la méglumine pour le DOTA-Gd. On obtient une solution liquide finale prête à administrer au patient.

Parmi les procédés industriels de préparation d'une poudre, le demandeur a désormais étudié de plus près le procédé d'ajustement dans le cas de la précipitation. En effet, le procédé par précipitation dans un solvant ou un mélange de solvants est avantageux pour une production industrielle.

L'homme du métier déduit directement du texte et des exemples de FR0851055 que l'on peut précipiter la solution obtenue à la fin de l'étape 1) ou à la fin de l'étape 3), dans un solvant organique approprié, de manière à obtenir une poudre qui pourra elle-même être remise en solution, cette solution étant injectée au patient. Tel que décrit dans la demande pendante FR0851055, les proportions dans la poudre du DOTA-Gd et du DOTA libre sont typiquement les mêmes entre d'une part la solution ajustée, et d'autre part la poudre.

Or, en réalisant des essais, le demandeur a désormais constaté que la précipitation posait un problème supplémentaire d'optimisation à l'échelle des lots industriels compte tenu des quantités et des solvants utilisés. Plus précisément, le demandeur s'est aperçu que la transformation par précipitation, de la solution liquide S obtenue à l'étape 1 ou à la fin de l'étape 3), du procédé décrit dans FR0851055, en une poudre P, s'accompagne typiquement d'une modification de proportions des entités, entre d'une part cette solution pharmaceutique S et d'autre part la poudre P, en ce qui concerne les entités :
- i) le chélate complexé (complexe DOTA-Gd par exemple)
- ii) le chélate libre (DOTA libre par exemple) tel que défini ci-dessus.

Pour maîtriser le procédé par précipitation, et en particulier pour s'assurer que la solution formulation pharmaceutique à administrer contienne la quantité d'excès de chélate libre cible (DOTA libre en particulier, de 0.002% à 0.4%), le demandeur a mis au point un procédé de préparation d'une formulation pharmaceutique de chélate de lanthanide (avantageusement de chélate macrocyclique de lanthanide) (avec ou sans excipient pharmaceutique tel que la méglumine) sous forme de poudre, cette poudre comprenant un excès de chélate libre mol/mol compris entre 0,002 et 0,4%, ledit procédé comprenant les étapes successives suivantes :
1) étape 1 : mélange du chélate (avantageusement du chélate macrocyclique) et du lanthanide pour obtenir la complexation du lanthanide par le chélate, la solution de complexation obtenue comprenant outre le complexe chélate-lanthanide, une quantité X1 d'excès de chélate libre
2) étape 2 : de préférence mesure de X1, et ajustement éventuel de X1 pour avoir X1 compris entre 0,002 et 0,4% mol/mol
3) étape 3 : précipitation de la solution de complexation obtenue à l'étape 1) ou à l'étape 2) dans un solvant organique de manière à obtenir une poudre de complexe chélate-lanthanide, la poudre contenant une quantité X2 d'excès de chélate libre,
4) étape 4 : ajustement éventuel de X2 de façon à obtenir :
   4.a) X2 est compris entre 0,002 et 0,4% mol/mol, et plus spécialement entre 0,02 et 0,3% mol/mol, très avantageusement entre 0,025 et 0,25% mol/mol, et
   4.b) X2 correspond à entre 0,1 et 5 fois X1, avantageusement entre 0,2 et 2 fois X1, notamment entre 0,5 à 1,5 fois X1
5) étape 5 : de préférence mesure de X2

Par chélate libre, on entend au sens de la présente invention tout chélate non complexé par un lanthanide ou un cation métallique, donc contrairement à l'art antérieur pas sous la forme d'un excipient de formule X[X',L] avec X, X' et L tels que définis ci-dessus.

L'étape 4) d'ajustement de X2 peut être mise en œuvre en ajoutant ou retirant du chélate libre par tout moyen approprié (résine par exemple), ou en ajoutant ou retirant du lanthanide.

Avantageusement X2 appartient aux gammes :
1) [0,1 - 0,95]^{∗}X1 (X2 correspond à entre 0,1 et 0,95 fois X1), encore plus avantageusement [0,2 - 0,95]^{∗}X1, particulièrement [0,5 - 0,95]^{∗}X1, plus particulièrement [0,6-0,9]^{∗}X1 ou
2) [1,05 - 5]^{∗}X1 (X2 correspond à entre 1,05 et 5 fois X1), encore plus avantageusement [1,05 - 2]^{∗}X1, en particulier [1,05-1,5]^{∗}X1, encore plus particulièrement [1,05-1,3]^{∗}X1.

Selon des réalisations, le solvant de l'étape 3) est choisi parmi : éthanol, méthanol, propanol, isopropanol, méthyle éthyle acétone, acétate d'éthyle, et leurs mélanges (y compris eau/solvant en toutes proportions) connus de l'homme du métier, avantageusement l'éthanol, le méthanol et le propanol.

Selon des réalisations, le solvant de l'étape 3) est utilisé selon un ratio de 5 à 20 volumes de solvant pour un volume de solution de complexation, avantageusement 10 à 20 volumes de solvant pour un volume de solution de complexation.

Selon des réalisations, le solvant de l'étape 3) est utilisé à une température comprise dans la gamme [0 - 80°C], notamment entre 30 et 70°C.

Selon des réalisations, on effectue une concentration partielle de la solution de complexation (par exemple par évaporation), avant de réaliser l'étape 3) de précipitation dans le solvant.

Selon des réalisations, le pH de la solution de complexation utilisé à l'étape 3) est compris entre 1 et 9.

Avantageusement on utilisera lors de l'étape 3) les conditions suivantes :
PH : [2 - 8]
[solvant éthanol] : 10 à 20 volumes pour un volume de solution de complexation Température : [0 ; 50] °C
Avantageusement, la poudre obtenue à l'étape 3) ou à l'étape 4) est remise en solution pour former une composition pharmaceutique sous forme liquide.

L'étape 1 utilise de préférence un excès de chélate, avantageusement de DOTA, par rapport aux proportions stoechiométriques de manière à obtenir une quantité X1 de chélate libre, avantageusement du DOTA libre.

L'étape 1 peut se faire en une seule étape ou éventuellement en plusieurs étapes successives par mesure et ajustement de manière à avoir une quantité X1 de chélate libre, avantageusement du DOTA libre (de préférence entre 0,002 et 0,4% mol/mol). Par exemple l'étape 1) comprend les sous-étapes successives suivantes :
1.1. complexation en solution
1.2. mesure du chélate, avantageusement du DOTA et/ou du lanthanide libre (avantageusement Gd)
1.3. addition de chélate, avantageusement de DOTA, de manière à complexer l'éventuel lanthanide libre (s'il y en a à la fin de l'étape 1) et à obtenir une quantité X1 de chélate libre, avantageusement de DOTA libre (de préférence entre 0,002 et 0,4% mol/mol).

Par «lanthanide libre », on entend, au sens de la présente divulgation, tout lanthanide non complexé et en particulier non complexé par le chélate.

Cette variante avec étape 1.1 à 1.3 est avantageuse car l'isolement d'une poudre permet de pouvoir purifier le produit.

Le chélate de lanthanide est le complexe DOTA-Gd.

Ainsi la maîtrise de l'étape de précipitation couplée à la mesure préalable de la quantité X1 de DOTA libre, fournit un ajustement du taux de DOTA, permettant de résoudre les problèmes de l'invention.

Typiquement on effectuera les mesures détaillées pour chaque lot de fabrication industrielle. On pourra aussi, à condition de totalement maîtriser la caractérisation des produits utilisés, construire une matrice de référence de calibrage obtenue grâce à l'étape (ou aux étapes) de mesure et ajustement décrite dans la demande.

Il est clair pour l'homme du métier que des sous-étapes d'optimisation peuvent être intercalées entre ces étapes principales.

La précipitation de l'étape 3) peut être réalisée en ajoutant le solvant à la solution de complexation, ou inversement en ajoutant la solution de complexation au solvant.

Selon une réalisation, la solution de l'étape 1 ne comprend pas le contre-ion positif (typiquement la méglumine ou l'ion Na+). L'étape 3 consistera ainsi à précipiter, non pas le DOTA-Gd sous forme de sel typiquement de sodium ou de méglumine (plus précisément [DOTA-Gd]⁻,Na⁺ ou [DOTA-Gd]⁻, CH2OH-(CHOH)4-CH2-N⁺-CH3), mais sa forme protonée [DOTA-Gd]⁻,H⁺, cette étape étant réalisée en milieu acide.

Selon une autre réalisation, d'ailleurs avantageuse et non décrite dans l'art antérieure, la solution de l'étape 1 comprend le contre-ion positif (méglumine en particulier) ; l'étape 3 consiste ainsi à précipiter selon des conditions appropriées (en particulier de pH et de quantité de solvant) le DOTA-Gd sel de méglumine [[DOTA-Gd]⁻, CH20H-(CHOH)4-CH2-N⁺-CH3]. Pour cela on utilise par exemple 10 à 20 volumes, par exemple 10 à 15 volumes, de solvant éthanol ou méthanol pour un volume de complexation, avantageusement à un pH entre 5 et 8 et une température 25-60°C. Cela permet d'utiliser un complexe DOTA-Gd sous forme de sel, plus stable que sous la forme protonée (du fait des constantes de complexation).

A cet effet l'invention concerne aussi un procédé tel que décrit précédemment dans la demande, la solution de complexation de l'étape 1 étant une solution de DOTA-Gd sel de méglumine ou sel de sodium, le complexe [DOTA-Gd] n'étant pas sous sa forme protonée dans cette solution. De manière très préférée la solution de complexation de l'étape 1 est une solution de [DOTA-Gd] sel de méglumine, ce qui a l'avantage par rapport à une solution de DOTA-Gd sel de sodium, lorsque l'on prépare une formulation méglumine avantageuse pour le patient du point de vue de la tolérance, de ne pas avoir à remplacer ensuite le sodium par la méglumine, opération qui est industriellement complexe.

Par ailleurs, on précise que le procédé par précipitation de la présente demande inclut le procédé décrit, et une variante par cristallisation qui se traduit par une modification des proportions des entités chélate complexé et chélate libre entre d'une part la composition pharmaceutique sous forme de solution liquide et d'autre part la composition pharmaceutique sous forme de poudre.

On décrit maintenant des exemples détaillés illustrant le procédé de préparation de formulations de chélate de lanthanide avec étape de précipitation, et plus particulièrement la préparation par précipitation de formulations comprenant un excès de chélate pour le DOTA.

Le tableau suivant indique un exemple des quantités utilisées pour la fabrication d'une solution de 100 litres de DOTA (quantité industrielle).

| *Composant* | *Quantité* |
|---|---|
| DOTA (1) | 20,100 kg (soit 0,497 M) |
| Oxyde de gadolinium (exprimé en produit anhydre) Gd₂O₃ | 9,135 kg (soit 0,252 M) |
| Méglumine (exprimée en produit anhydre) | 9,215 kg |
| Solution d'ajustage de DOTA à 15 % (m/v) qsp taux de DOTA libre | 15-35 mg pour 100 ml |
| Solution de méglumine à 2 N qsp pH=6 ,8-7,4 à 20°C | |
| Eau ppi ... qsp | 100 litres |

| | |
|---|---|
| (1) acide 1,4,7,10-tétraazacyclododécane-N,N',N",N'"-tétraacétique | |

### Etape 1.1 : complexation en solution

Le DOTA et l'oxyde de gadolinium sont dispersés dans l'eau ppi à environ 80°C. L'oxyde de gadolinium en présence de DOTA forme un complexe-acide soluble dans l'eau.

Dans une cuve de fabrication de 100 litres, on introduit 40 litres d'eau ppi à 80°C, on met en route l'injection d'azote, puis on incorpore sous agitation les 20,100 kg de DOTA et les 9,135 kg d'oxyde de gadolinium. La complexation est réalisée à pH inférieur à 6.

### Etapes 1.2. et 1.3. : ajustement des entités libres

L'ajustement de la solution est effectué avantageusement par oxyde de gadolinium ou de DOTA.

A l'issue de l'étape 1.1, les étapes suivantes ont lieu :
1.2. prélèvement d'un échantillon et dosage du gadolinium libre,
1.3. ajout d'une solution d'ajustage de DOTA qsp à une quantité de 15-35 mg pour 100 ml

On obtient une solution de complexation S avec un taux X1 de chélate libre que l'on peut mesurer (étape 2). On peut aussi éliminer tout gadolinium résiduel grâce à une résine chélex par exemple préalablement rincée à l'eau (une quantification du gadolinium libre peut être réalisée par un dosage colorimétrique avec de l'Arsenazo (III) avant cette élimination, et éventuellement aussi après). Pour cela, le mélange réactionnel peut être ramené à pH 5 (la résine est plus efficace). L'ensemble est laissé 2 heures sous agitation à température ambiante. Le pH remonte entre 6,5 et 7. La résine est éliminée par filtration.

La valeur X1 (en pourcentage mol/mol) est selon les lots par exemple comprise entre 0,005 et 0,1%, notamment 0,01 à 0,05% mol/mol de DOTA libre (DOTA non complexé par le Gd, ni par des cations tels que Ca2+).

### Etape 3 : précipitation de la solution de complexation

On peut additionner directement la solution obtenue à l'étape 2 au solvant, mais on réalise typiquement pour réduire les quantités de solution à traiter, une concentration sous vide préalable sans modification significative du taux de DOTA libre.

La solution aqueuse de DOTA-Gd est concentrée sous vide à une température inférieure à 80°C (par exemple entre 20 et 60°C, par exemple 50 à 70 °C) jusqu'à une concentration de 0,5 kg (+/- 0,2 kg) par kg de solution. La solution est refroidie à température ambiante.

Pour une quantité de 1 Kg de solution concentrée, une solution de 5 à 20 litres (de préférence 10 à 20 litres, et typiquement 20 litres) d'éthanol est ajoutée dans 1kg de solution concentrée. Le DOTA-Gd précipité obtenu est filtré, puis repris par 3-4 litres d'éthanol.

Rapportée à une quantité industrielle de 100 Kg de solution de DOTA-Gd, on utilisera ainsi par exemple une quantité de l'ordre de 2000 litres d'éthanol pour effectuer la précipitation.

Le produit obtenu est séché sous vide à une température inférieure à 80°C.

On utilise par exemple l'éthanol dénaturé suivant : éthanol 95%, mélange acétate d'éthyle et isopropanol 5%.

Le composé obtenu par précipitation (poudre) contient une quantité X2 de chélate libre. Selon les solvants utilisés on obtient une quantité X2 (en pourcentage mol/mol) variant selon les essais par exemple entre 0,02% et 0,15% mol/mol.

Par ailleurs les résultats ne sont pas toujours identiques selon que l'on réalise la précipitation à partir d'une solution de DOTA-Gd (avec excès X1 de DOTA libre) avec méglumine, ou d'une solution de DOTA-Gd (avec excès X1 de DOTA libre) sans méglumine, la méglumine étant à prendre en compte du point de vue de la solubilisation.

### Etape 2bis éventuelle après l'étape 2 et avant l'étape 3 : refroidissement

La solution finale de l'étape 2 est refroidie à 30°C, par exemple par circulation d'eau froide dans la double enveloppe de la cuve
Etape 2ter éventuelle après l'étape 2bis et avant l'étape 3 : ajustement du pH et de la masse volumique
La fonction acide du complexe formé est salifiée par de la méglumine et le pH à 20°C est ajusté à 6 ,8 - 7,4. La concentration est ajustée par addition d'eau ppi.

On introduit dans la cuve de fabrication :
9,125 kg de méglumine
et une solution de méglumine à pH = 7,3 -7,4 à 2N
et de l'eau ppi qsp

### Etape 5 après l'étape 3 ou 4 :

Le produit précipité en poudre obtenu est remis en solution aqueuse pharmaceutique (comprenant typiquement la méglumine) ensuite filtrée puis mise en flacons stérilisés typiquement par autoclavage.

## Revendications

1. Procédé de préparation d'une formulation pharmaceutique liquide de chélate de lanthanide, comprenant un excès de chélate libre mol/mol compris entre 0,002 et 0,4%, le chélate étant du DOTA, le lanthanide étant du Gadolinium, le chélate de lanthanide étant le complexe DOTA-Gd, et ledit procédé comprenant les étapes successives suivantes :
1) étape 1 : mélange du chélate et du lanthanide pour obtenir la complexation du lanthanide par le chélate, la solution de complexation obtenue comprenant outre le complexe chélate-lanthanide, une quantité X1 d'excès de chélate libre,
2) étape 2 : de préférence mesure de X1 et ajustement éventuel de X1 pour avoir X1 compris entre 0,002 et 0,4% mol/mol,
3) étape 3 : précipitation ou cristallisation de la solution de complexation obtenue à l'étape 1) ou à l'étape 2) dans un solvant organique choisi parmi l'éthanol, le méthanol, le propanol, l'isopropanol, la méthyle éthyle acétone, l'acétate d'éthyle, et leurs mélanges y compris aqueux, selon un ratio de 5 à 20 volumes de solvant pour un volume de solution de complexation, à une température comprise dans la gamme [0 ; 80°C], de manière à obtenir une poudre de complexe chélate-lanthanide, la poudre contenant une quantité X2 d'excès de chélate libre,
4) étape 4 : ajustement de X2 de façon à obtenir :
4.a) X2 compris entre 0,002 et 0,4%, et plus spécialement entre 0,02 et 0,3% mol/mol, très avantageusement entre 0,025 et 0,25% mol/mol, et
4.b) X2 correspondant à entre 0,2 et 2 fois X1, notamment entre 0,5 et 1,5 fois X1, X2 appartenant avantageusement à la gamme : [0,5 - 0,95]^{∗}X1, ou à la gamme [1,05-1,3]^{∗}X1,
5) étape 5 : de préférence mesure de X2,
6) étape 6 : mise en solution de la poudre obtenue à l'étape 4), menant à une formulation pharmaceutique liquide de chélate de lanthanide, comprenant un excès de chélate libre mol/mol compris entre 0,002 et 0,4%.

2. Procédé selon la revendication 1, **caractérisé en ce que** l'étape 3) est une précipitation de la solution de complexation obtenue à l'étape 1) ou à l'étape 2) dans un solvant organique choisi parmi l'éthanol, le méthanol, le propanol, l'isopropanol, la méthyle éthyle acétone, l'acétate d'éthyle, et leurs mélanges y compris aqueux, selon un ratio de 5 à 20 volumes de solvant pour un volume de solution de complexation, à une température comprise dans la gamme [0 ; 80°C], de manière à obtenir une poudre de complexe chélate-lanthanide, la poudre contenant une quantité X2 d'excès de chélate libre.

3. Procédé selon la revendication 1 ou 2, **caractérisé en ce que** l'étape 1) est réalisée en une seule étape ou en plusieurs étapes successives par mesure et ajustement, de manière à avoir une quantité X1 de chélate libre, l'étape 1) comprenant avantageusement les sous-étapes successives suivantes :
1.1) complexation en solution du lanthanide par le chélate,
1.2) mesure du chélate et/ou du lanthanide libre,
1.3) addition de chélate libre, de manière à complexer le lanthanide libre s'il en reste à la fin de l'étape 1.1), et à obtenir une quantité X1 de chélate libre.

4. Procédé selon l'une quelconque des revendications 1 à 3, **caractérisé en ce que** le solvant de l'étape 3) est choisi parmi l'éthanol, le méthanol, le propanol.

5. Procédé selon l'une quelconque des revendications 1 à 4, **caractérisé en ce que** l'étape 3) est conduite à un pH compris entre 1 et 9, de préférence entre 2 et 8.

6. Procédé selon l'une quelconque des revendications 1 à 5, **caractérisé en ce que** le solvant de l'étape 3) est utilisé selon un ratio de 10 à 20 volumes de solvant pour un volume de solution de complexation.

7. Procédé selon l'une quelconque des revendications 1 à 6, **caractérisé en ce qu'**on effectue une concentration partielle de la solution de complexation avant de réaliser l'étape 3) de précipitation du solvant.

8. Procédé selon l'une quelconque des revendications 1 à 7, **caractérisé en ce que** le solvant de l'étape 3) est utilisé à une température comprise dans la gamme [30 ; 70°C].

9. Procédé selon l'une quelconque des revendications 1 à 8 **caractérisé en ce que**, la solution de complexation de l'étape 1 est une solution de [DOTA-Gd] sel de méglumine.

10. Procédé selon la revendication 9, **caractérisé en ce que** la méglumine est ajoutée à l'étape 1), et **en ce que** l'étape 3) consiste en une précipitation, et à précipiter le sel de méglumine de DOTA-Gd.

11. Procédé selon la revendication 10, **caractérisé en ce que** l'étape 3) est effectuée dans 10 à 15 volumes d'éthanol ou de méthanol pour un volume de complexation, à un pH entre 5 et 8 et à une température comprise entre 25 et 60°C.

12. Procédé selon la revendication 1, **caractérisé en ce que** l'étape 1) conduit au complexe de DOTA-Gd sous sa forme protonée.

13. Procédé selon la revendication 12, **caractérisé en ce que** l'étape 3) consiste en une précipitation, et à précipiter le DOTA-Gd sous sa forme protonée en milieu acide.

## Patentansprüche

1. Verfahren zur Herstellung einer flüssigen pharmazeutischen Formulierung von Lanthanoid-Chelat, umfassend einen Überschuss an freiem Chelat-Mol/Mol zwischen 0,002 und 0,4 %, wobei das Chelat DOTA ist, Lanthanoid Gadolinium ist, Lanthanoid-Chelat der DOTA-Gd-Komplex ist, und das Verfahren die folgenden Schritte nacheinander umfasst:
1) Schritt 1: Mischen von Chelat und Lanthanoid, um die Komplexierung von Lanthanoid durch Chelat zu erzielen, wobei die erhaltene Komplexierungslösung außer dem Lanthanoid-Chelat-Komplex eine Menge X1 an überschüssigem freiem Chelat umfasst,
2) Schritt 2: vorzugsweise Messen von X1 und eventuelle Einstellung von X1, damit X1 zwischen 0,002 und 0,4 % Mol/Mol liegt,
3) Schritt 3: Ausfällung oder Kristallisation der in Schritt 1) oder Schritt 2) erhaltenen Komplexierungslösung in einem organischen Lösungsmittel, das unter Ethanol, Methanol, Propanol, Isopropanol, Methylethylaceton, Ethylacetat und Mischungen davon einschließlich wässriger in einem Verhältnis von 5 bis 20 Teilen Lösungsmittel auf ein Teil Komplexierungslösung bei einer Temperatur innerhalb des Bereichs [0; 80 °C] ausgewählt wird, um ein Lanthanoid-Chelat-Komplexpulver zu erhalten, wobei das Pulver eine Menge X2 an überschüssigem freiem Chelat enthält,
4) Schritt 4: Einstellen von X2, um Folgendes zu erhalten:
4.a) X2 zwischen 0,002 und 0,4 % und spezieller zwischen 0,02 und 0,3 % Mol/Mol, sehr vorteilhafterweise zwischen 0,025 und 0,25 % Mol/Mol, und
4.b) wobei X2 zwischen 0,2 und 2 mal X1 entspricht, insbesondere zwischen 0,5 und 1,5 mal X1, wobei X2 vorteilhafterweise folgendem Bereich entspricht: [0,5-0,95]^{∗}X1 oder dem Bereich [1,05-1,3]^{∗}X1,
5) Schritt 5: vorzugsweise Messen von X2,
6) Schritt 6: Lösen des in Schritt 4) erhaltenen Pulvers, was zu einer flüssigen pharmazeutischen Formulierung von Lanthanoid-Chelat führt, umfassend einen Überschuss an freiem Chelat Mol/Mol zwischen 0,002 und 0,4 %.

2. Verfahren nach Anspruch 1, **dadurch gekennzeichnet, dass** Schritt 3) eine Ausfällung der in Schritt 1) oder Schritt 2) erhaltenen Komplexierungslösung in einem organischen Lösungsmittel ist, das unter Ethanol, Methanol, Propanol, Isopropanol, Methylethylaceton, Ethylacetat und Mischungen davon, einschließlich wässriger, in einem Verhältnis von 5 bis 20 Teilen Lösungsmittel auf ein Teil Komplexierungslösung bei einer Temperatur im Bereich [0; 80 °C] ausgewählt wird, um ein Lanthanoid-Chelat-Komplexpulver zu erhalten, wobei das Pulver eine Menge X2 an überschüssigem freiem Chelat enthält.

3. Verfahren nach Anspruch 1 oder 2, **dadurch gekennzeichnet, dass** Schritt 1) in einem einzigen Schritt oder in mehreren Schritten nacheinander pro Messung und Einstellung durchgeführt wird, um eine Menge X1 an freiem Chelat zu erhalten, wobei Schritt 1) vorzugsweise die folgenden Teilschritte nacheinander umfasst:
1.1) Komplexierung in einer Lösung des Lanthanoids durch Chelat,
1.2) Messen des Chelats und/oder des freien Lanthanoids,
1.3) Zusatz von freiem Chelat, um das freie Lanthanoid zu komplexieren, wenn am Ende des Schritts 1.1) welches übrig bleibt, und eine Menge X1 an freiem Chelat zu erhalten.

4. Verfahren nach einem der Ansprüche 1 bis 3, **dadurch gekennzeichnet, dass** das Lösungsmittel von Schritt 3) unter Ethanol, Methanol, Propanol ausgewählt wird.

5. Verfahren nach einem der Ansprüche 1 bis 4, **dadurch gekennzeichnet, dass** Schritt 3) bei einem pH-Wert zwischen 1 und 9, vorzugsweise zwischen 2 und 8, durchgeführt wird.

6. Verfahren nach einem der Ansprüche 1 bis 5, **dadurch gekennzeichnet, dass** das Lösungsmittel aus Schritt 3) in einem Verhältnis von 10 bis 20 Teilen Lösungsmittel auf ein Teil Komplexierungslösung verwendet wird.

7. Verfahren nach einem der Ansprüche 1 bis 6, **dadurch gekennzeichnet, dass** eine Teilkonzentration der Komplexierungslösung durchgeführt wird, bevor Schritt 3) der Ausfällung des Lösungsmittels durchgeführt wird.

8. Verfahren nach einem der Ansprüche 1 bis 7, **dadurch gekennzeichnet, dass** das Lösungsmittel aus Schritt 3) bei einer Temperatur im Bereich [30; 70 °C] verwendet wird.

9. Verfahren nach einem der Ansprüche 1 bis 8, **dadurch gekennzeichnet, dass** die Komplexierungslösung von Schritt 1 eine Lösung von [DOTA-Gd] Megluminsalz ist.

10. Verfahren nach Anspruch 9, **dadurch gekennzeichnet, dass** Meglumin in Schritt 1) zugesetzt wird, und dass der Schritt 3) aus einer Ausfällung und aus dem Fällen des Megluminsalzes von DOTA-Gd besteht.

11. Verfahren nach Anspruch 10, **dadurch gekennzeichnet, dass** Schritt 3) in 10 bis 15 Teilen Ethanol oder Methanol auf ein Komplexierungsteil bei einem pH-Wert zwischen 5 und 8 und einer Temperatur zwischen 25 und 60 °C durchgeführt wird.

12. Verfahren nach Anspruch 1,
**dadurch gekennzeichnet, dass** Schritt 1) zum DOTA-Gd-Komplex in seiner protonierten Form führt.

13. Verfahren nach Anspruch 12, **dadurch gekennzeichnet, dass** Schritt 3) aus einer Ausfällung und aus dem Fällen des DOTA-Gd in seiner protonierten Form in saurem Milieu besteht.

## Claims

1. Preparation method for a liquid pharmaceutical formulation of lanthanide chelate comprising a mol/mol excess of free chelate comprised between 0.002 and 0.4%, the chelate being DOTA, the lanthanide being Gadolinium, the lanthanide chelate being the DOTA-Gd complex, and said method comprising the following successive steps:
1) Step 1: mixture of the chelate and the lanthanide to obtain complexation of the lanthanide by the chelate, the complexation solution obtained comprising, in addition to the chelate-lanthanide complex, a quantity X1 of excess free chelate,
2) Step 2: preferably measurement of X1 and possible adjustment of X1 to have X1 comprised between 0.002 and 0.4% mol/mol,
3) Step 3: precipitation or crystallization of the complexation solution obtained in step 1) or step 2) in an organic solvent chosen from among ethanol, methanol, propanol, isopropanol, methyl ethyl acetone, ethyl acetate, and mixtures thereof, including aqueous, according to a ratio of 5 to 20 volumes of solvent for one volume of complexation solution, at a temperature comprised within the range [0; 80°C],so as to obtain a chelate-lanthanide complex powder, the powder containing a quantity X2 of excess free chelate,
4) Step 4: adjustment of X2 so as to obtain:
4.a) X2 comprised between 0.002 and 0.4%, and more especially between 0.02 and 0.3% mol/mol, very advantageously between 0.025 and 0.25% mol/mol, and
4.b) X2 corresponding to between 0.2 and 2 times X1, notably between 0.5 and 1.5 times X1, X2 advantageously belonging to the range: [0.5 - 0.95]^{∗}X1, or the range [1.05-1.3]^{∗}X1,
5) Step 5: preferably measurement of X2,
6) Step 6: putting the powder obtained in step 4) in solution, leading to a liquid pharmaceutical formulation of lanthanide chelate comprising a mol/mol excess of free chelate comprised between 0.002 and 0.4%.

2. Method according to claim 1, **characterized in that** step 3) is a precipitation of the complexation solution obtained in step 1) or step 2) in an organic solvent chosen from among ethanol, methanol, propanol, isopropanol, methyl ethyl acetone, ethyl acetate, and mixtures thereof, including aqueous, according to a ratio of 5 to 20 volumes of solvent for one volume of complexation solution, at a temperature comprised within the range [0; 80°C],so as to obtain a chelate-lanthanide complex powder, the powder containing a quantity X2 of excess free chelate.

3. Method according to claim 1 or 2, **characterized in that** step 1) is performed in a single step or in several successive steps by measurement and adjustment, so as to have a quantity X1 of free chelate, step 1) advantageously comprising the following successive substeps:
1.1) complexation in lanthanide solution by the chelate,
1.2) measurement of the free chelate and/or lanthanide,
1.3) addition of free chelate, so as to complex the free lanthanide if there is any remaining at the end of step 1.1), and obtaining a quantity X1 of free chelate.

4. Method according to any one of claims 1 to 3, **characterized in that** the solvent of step 3) is chosen from among ethanol, methanol, propanol.

5. Method according to any one of claims 1 to 4, **characterized in that** step 3) is conducted at a pH comprised between 1 and 9, preferably between 2 and 8.

6. Method according to anyone of claims 1 to 5, **characterized in that** the solvent of step 3) is used according to a ratio of 10 to 20 volumes of solvent for one volume of complexation solution.

7. Method according to any one of claims 1 to 6, **characterized in that** a partial concentration of the complexation solution is conducted before performing step 3) of solvent precipitation.

8. Method according to any one of claims 1 to 7, **characterized in that** the solvent of step 3) is used at a temperature comprised in within the range [30; 70°C].

9. Method according to any one of claims 1 to 8, **characterized in that** the complexation solution of step 1 is a solution of [DOTA-Gd] meglumine salt

10. Method according to claim 9, **characterized in that** meglumine is added at step 1), and **in that** step 3) consists of precipitation, and precipitating the DOTA-Gd meglumine salt.

11. Method according to claim 10, **characterized in that** step 3) is performed in 10 to 15 volumes of ethanol or methanol for one volume of complexation, at a pH between 5 and 8 and a temperature comprised between 25 and 60°C.

12. Method according to claim 1
**characterized in that** step 1) leads to a DOTA-Gd complex in the protonated form.

13. Method according to claim 12, **characterized in that** step 3) consists of a precipitation, and precipitating DOTA-Gd in its protonated form in acid medium.
